# EUROPEAN PATENT SPECIFICATION

(11) **EP 2 111 166 B1**
(45) Date of publication and mention of the grant of the patent: **03.03.2021**
(21) Application number: 08713760.0
(22) Date of filing: 15.01.2008
(51) Int. Cl.: A61B 8/14, A61B 17/29, A61B 17/32

(54) **ULTRASONIC DEVICE FOR CUTTING AND COAGULATING**
SCHNEID- UND GERINNUNGSULTRASCHALLGERÄT
DISPOSITIF À ULTRASONS FACILITANT LA COUPE ET LA COAGULATION

(30) Priority: 16.01.2007 US 885086 P; 28.08.2007 US 968357 P
(43) Date of publication of application: 28.10.2009
(73) Proprietor: Ethicon LLC, 00969 Guaynabo (PR)
(72) Inventor: VOEGELE, Aaron C., Loveland, Ohio 45140 (US); PRICE, Daniel W., Loveland, Ohio 45140 (US); WEED, III, John A., Monroe, Ohio 45050 (US); RUPP, Kip M., New Richmond, Ohio 45157 (US); NEUROHR, Mark A., Newport, Kentucky 41071 (US); KROSCHER, Nicholas I., Mason, Ohio 45040 (US); WOODRUFF, Scott A., Gahanna, Ohio 43230 (US); EICHMANN, Stephen E., Cincinnati, Ohio 45249 (US); MCFARLAND, Terry A., Burlington, Kentucky 41005 (US); LOPES, Tracy D., Mason, Ohio 45040 (US)
(74) Representative: Tunstall, Christopher Stephen
(86) International application number: PCT/US2008/051061
(87) International publication number: WO 2008/089174

(56) References cited:
- GB-A- 2 380 679
- US-A- 5 221 282
- US-A- 5 358 505
- US-A- 5 399 930
- US-A1- 2002 049 464
- US-A1- 2002 049 464
- US-A1- 2003 069 576
- US-A1- 2003 069 576
- US-A1- 2003 088 235
- US-A1- 2003 212 392
- US-A1- 2005 049 525
- US-A1- 2005 267 502
- US-A1- 2005 267 502
- US-A1- 2006 069 314
- US-A1- 2006 079 876
- US-A1- 2006 079 879

## Description

### Field of the Invention

The present invention generally relates to ultrasonic surgical systems and, more particularly, to an ultrasonic device that allows surgeons to perform cutting, coagulation, and fine dissection required in fine and delicate surgical procedures such as plastic surgery.

### Background of the Invention

Ultrasonic surgical instruments are finding increasingly widespread applications in surgical procedures by virtue of the unique performance characteristics of such instruments. Depending upon specific instrument configurations and operational parameters, ultrasonic surgical instruments can provide substantially simultaneous cutting of tissue and homeostasis by coagulation, desirably minimizing patient trauma. The cutting action is typically realized by an end-effector, or blade tip, at the distal end of the instrument, which transmits ultrasonic energy to tissue brought into contact with the end-effector. Ultrasonic instruments of this nature can be configured for open surgical use, laparoscopic or endoscopic surgical procedures including robotic-assisted procedures.

Performing an average plastic surgery procedure (e.g. abdominoplasty, breast reconstruction/reduction, and face lift) involves significant recovery time for the patient and risk of post-operative complications such as seroma and hematoma. The recovery time includes additional office visits post-operatively, affecting patient satisfaction and decreasing the amount of time a surgeon is available for surgery. Advanced energy instruments (in lieu of traditional monopolar electrosurgery - "bovie") can provide a less complicated recovery experience and potentially shorten the post-operative recovery time. However, the advanced energy instruments currently available are not designed specifically for plastic surgery procedures. They lack the comfort and versatility required for such procedures.

For example, present energy instruments are available only in fixed lengths. This is a problem for many plastic surgery procedures because the surgeon prefers to have a short blade at the beginning of a procedure for superficial work and a longer blade later during the procedure to obtain deeper access to tissue. With current instruments, the surgeon is required to switch instruments during the procedure, which is both time and cost prohibitive.

Some surgical instruments utilize ultrasonic energy for both precise cutting and controlled coagulation. Ultrasonic energy cuts and coagulates by using lower temperatures than those used by electrosurgery. Vibrating at high frequencies (e.g. 55,500 times per second), the ultrasonic blade denatures protein in the tissue to form a sticky coagulum. Pressure exerted on tissue with the blade surface collapses blood vessels and allows the coagulum to form a hemostatic seal. The precision of cutting and coagulation is controlled by the surgeon's technique and adjusting the power level, blade edge, tissue traction and blade pressure.

Some current designs of ultrasonic surgical devices utilize a foot pedal to energize the surgical instrument. The surgeon operates the foot pedal to activate a generator that provides energy that is transmitted to the cutting blade for cutting and coagulating tissue while simultaneously applying pressure to the handle to press tissue against the blade. Key drawbacks with this type of instrument activation include the loss of focus on the surgical field while the surgeon searches for the foot pedal, the foot pedal getting in the way of the surgeon's movement during a procedure and surgeon leg fatigue during long cases.

Document US2003/088235 A1 discloses liposuction devices. The devices include an evacuation tube surrounding an ultrasonic probe, including the distal end thereof to define a liquefaction and/or rupture chamber. Containing the ultrasonic probe within the evacuation tube avoids any possible contact with surrounding tissue other than tissue that gets drawn into the liquefaction and/or rupture chamber. The size of the liquefaction and/or rupture chamber may be variable by adjustment of the axial position of the evacuation tube relative to the ultrasonic probe, or by selection of evacuation tubes of the desired length..

It would be desirable to provide an ultrasonic surgical instrument that overcomes some of the deficiencies of current instruments. The ultrasonic surgical instrument described herein overcomes those deficiencies.

### Summary of the Invention

Claim 1 defines the invention and the dependent claims define optional features. An ultrasonic surgical instrument assembly embodying the principles of the present invention is configured to permit selective dissection, cutting, coagulation and clamping of tissue during surgical procedures.

A first expression of a first embodiment of an ultrasonic surgical instrument is a housing configured to accept a transducer and further defining a longitudinal axis; a first switch positioned on the housing for actuation by one or more fingers of a user and further electrically connected to a generator for providing an electrical signal to the generator for controlling a first level of ultrasonic energy delivered by the transducer. A second expression of the first embodiment of an ultrasonic surgical instrument is for a second switch positioned on the housing for actuation by one or more fingers of a user and further electrically connected to a generator for providing an electrical signal to the generator for controlling a second level of ultrasonic energy delivered by the transducer.

A first expression of a second embodiment of an ultrasonic surgical instrument is a blade extending along a longitudinal axis of the housing and configured to translate or telescope along the longitudinal axis. Such a feature allows the user to have one instrument with multiple blade lengths. The distance of the activation buttons adjusts with respect to the distal end of the blade and thereby provides precise control in the short blade position and deep access in the longer positions. This also allows for fewer instrument exchanges to reduce procedure time.

A second expression of the second embodiment is a sheath enclosing the blade and the sheath configured to translate along a longitudinal axis.

A third expression of the second embodiment is a sheath enclosing the blade and the blade configured to rotate with respect to the housing.

A first expression of a third embodiment of an ultrasonic surgical instrument is a locking mechanism for preventing the blade and/or sheath from translating along the longitudinal axis.

A second expression of the third embodiment is a locking mechanism for preventing the blade from rotating with respect to the housing.

### Brief Description of the Figures

The novel features of the invention are set forth with particularity in the appended claims. The invention itself, however, may best be understood by reference to the following description, taken in conjunction with the accompanying drawings in which:
FIGURE 1A is a perspective exploded assembly view illustrating an embodiment of an ultrasonic surgical instrument;
FIGURE 1B-C are alternate perspective views of the assembled instrument of FIG. 1A;
FIGURE 2A is a partial cutaway perspective view of one embodiment of the invention having multiple switches to support blade translation with the end effector in a most distal position;
FIGURE 2B is a partial cutaway perspective view of one embodiment of the invention using multiple switches to support blade translation with the end effector in a most proximal location;
FIGURE 2C is an exploded cutaway perspective view illustrating one embodiment of the rotation mechanism using detent features;
FIGURE 2D is an electrical schematic of an alternate embodiment of the hand switch circuit having multiple switches;
FIGURE 2E is a cutaway view of one embodiment of the translation mechanism using a spiral flex circuit with the end effector in a distal location;
FIGURE 2F is a cutaway view of one embodiment of the translation mechanism using a spiral flex circuit with the end effector in a proximal location;
FIGURE 2G is an enlarged elevation view of the one embodiment of the electrical connection with a spiral flex circuit;
FIGURE 2H is an enlarged elevation view of one embodiment of the invention illustrating one embodiment of the rotation mechanism using detent features;
FIGURE 2I is a cutaway perspective view of one embodiment of the translation mechanism using an electrical rail connector with the end effector in a proximal location;
FIGURE 2J is an enlarged view of an alternate embodiment of the electrical connection utilizing an electrical rail;
FIGURE 2K is a cutaway perspective view of one embodiment of the translation mechanism using an electrical rail connector with the end effector in a distal location;
FIGURE 2L is an enlarged cutaway perspective view of one embodiment of rotational mechanism utilizing circular detent features;
FIGURE 2M is an enlarged cutaway perspective view of one embodiment of the electrical connection using concentric cylinders with bushings;
FIGURE 2N is an exploded assembly view illustrating one embodiment of the electrical connection showing the concentric cylinders with bushings;
FIGURE 3A is an exploded perspective view of an alternate embodiment of the translation and rotation mechanism utilizing a helix on the blade sheath;
FIGURE 3B is a cutaway perspective view of one embodiment of the translation and rotation mechanism utilizing a friction lock;
FIGURES 3C-D are an elevation and side view, respectively, view of one embodiment of the friction lock knob;
FIGURE 3D is a side view of one embodiment of the friction lock knob;
FIGURE 3E is a side and cross sectional view of an alternate embodiment of the friction lock knob;
FIGURE 3F is an exploded view of an alternate embodiment of the translation and rotation mechanism utilizing a friction lock;
FIGURE 4A is an elevation view of an alternate embodiment illustrating a finger pad for coagulating;
FIGURE 4B is a side view of an alternate embodiment illustrating a finger pad for coagulating;
FIGURE 4C is a perspective view of an alternate embodiment illustrating a pad on a stick for coagulating;
FIGURE 5A is a cutaway elevation view of one embodiment of the blade and pin assembly;
FIGURE 5B is an exploded assembly view of one embodiment of the blade and pin assembly;
FIGURE 6 is a perspective view of an alternate embodiment utilizing a lighting system;
FIGURE 7A is a perspective view of one embodiment containing a haptic ring activation assembly;
FIGURE 7B is an exploded perspective view of the haptic ring activation assembly;
FIGURE 8A is an elevation view of one embodiment of a counterbalance mechanism;
FIGURE 8B is an elevation view of one embodiment of a counterbalance mechanism;
FIGURE 8C is an elevation view an alternate embodiment of the counterbalance mechanism with movable weights;
FIGURE 8D is an elevation view of an alternate embodiment of the counterbalance mechanism containing movable weights and a gear system;
FIGURE 9A is a perspective view of an alternate embodiment of the invention having slidable activation buttons attached through a magnetic connection;
FIGURE 9B is a cutaway elevation view of one embodiment of the invention showing the magnetic rail connections;
FIGURE 9C is an enlarged perspective view of one embodiment of the activation button assembly that is attached through a magnetic connection;
FIGURE 10 is an electrical schematic of a hand switch circuit;
FIGURE 11A is a perspective view of a hand wrench in accordance with the present invention;
FIGURE 11B is an elevation view of the hand wrench of FIG. 11A;
FIGURE 11C is a cross sectional end view of the distal end of a hand wrench depicting cantilever arm and teeth geometry; and
FIGURE 11D is a cross sectional view of an adaptor depicting spline gear geometry.

### Detailed Description of the Invention

In this specifications the following non-SI units are used, which may be converted to the respective SI or metric unit according to the following conversion table: 1 inch = 25.4 mm; 1 Kg = 2.2 Ibs.Before
explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practiced or carried out in various ways. Further, unless otherwise indicated, the terms and expressions employed herein have been chosen for the purpose of describing the illustrative embodiments of the present invention for the convenience of the reader and are not for the purpose of limiting the invention.

Further, it is understood that any one or more of the following-described embodiments, expressions of embodiments, examples, etc. can be combined with any one or more of the other following-described embodiments, expressions of embodiments, examples, etc.

The present invention is particularly directed to an improved ultrasonic surgical instrument, which is configured for effecting tissue dissecting, cutting and/or coagulation during surgical procedures, including delicate surgical procedures, such as plastic surgery. The present apparatus is configured for use in open surgical procedures, but has applications in other types of surgery, such as laparoscopic. Versatile use is facilitated by selective use of ultrasonic energy. When ultrasonic components of the apparatus are inactive, tissue can be manipulated, as desired, without tissue cutting or damage. When the ultrasonic components are activated the ultrasonic energy provides for both tissue cutting and coagulation.

Further, the present invention is disclosed in terms of a blade-only instrument. This feature is not intended to be limiting, as the embodiments disclosed herein have equal application in clamp coagulator instruments as are exemplary disclosed in U.S. patent nos. 5,873,873 and 6,773,444.

As will become apparent from the following description, the present surgical apparatus is particularly configured for disposable use by virtue of its straightforward construction. As such, it is contemplated that the apparatus be used in association with an ultrasonic generator unit of a surgical system, whereby ultrasonic energy from the generator unit provides the desired ultrasonic actuation for the present surgical instrument. It will be appreciated that surgical instrument embodying the principles of the present invention can be configured for non-disposable or multiple use, and non-detachably integrated with an associated ultrasonic generator unit. However, detachable connection of the present surgical instrument with an associated ultrasonic generator unit is presently preferred for single-patient use of the apparatus.

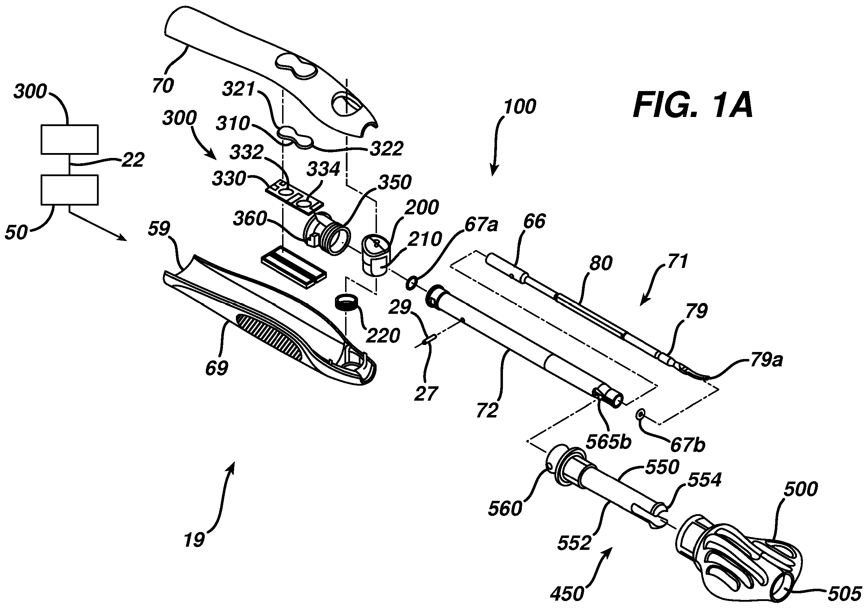

With specific reference now to Figs. 1A-C, an embodiment of a surgical system 19, including an ultrasonic surgical instrument 100 in accordance with the present invention is illustrated. The surgical system 19 includes an ultrasonic generator 300 connected to an ultrasonic transducer 50 via cable 22, and an ultrasonic surgical instrument 100. It will be noted that, in many applications, the ultrasonic transducer 50 is also referred to as a "hand piece assembly" or "handpiece" because the surgical instrument of the surgical system 19 is configured such that a surgeon may grasp and manipulate the ultrasonic transducer 50 during various procedures and operations. A suitable generator is the GEN04 (also referred to as Generator 300) sold by Ethicon Endo-Surgery, Inc. of Cincinnati, Ohio. A suitable transducer is disclosed in co-pending U.S. patent application filed on October 10, 2006, serial no. 11/545,784, entitled MEDICAL ULTRASOUND SYSTEM AND HANDPIECE AND METHODS FOR MAKING AND TUNING.

Ultrasonic transducer 50 and an ultrasonic waveguide 80 together provide an acoustic assembly of the present surgical system 19, with the acoustic assembly providing ultrasonic energy for surgical procedures when powered by generator 300. The acoustic assembly of surgical instrument 100 generally includes a first acoustic portion and a second acoustic portion. In the present embodiment, the first acoustic portion comprises the ultrasonically active portions of ultrasonic transducer 50, and the second acoustic portion comprises the ultrasonically active portions of transmission assembly 71. Further, in the present embodiment, the distal end of the first acoustic portion is operatively coupled to the proximal end of the second acoustic portion by, for example, a threaded connection.

The ultrasonic surgical instrument 100 includes a multi-piece handle assembly 68 adapted to isolate the operator from the vibrations of the acoustic assembly contained within transducer 50. The handle assembly 68 can be shaped to be held by a user in a conventional manner, but it is contemplated that the present ultrasonic surgical instrument 100 principally be grasped and manipulated in a pencil-like arrangement provided by a handle assembly of the instrument, as will be described. While a multi-piece handle assembly 68 is illustrated, the handle assembly 68 may comprise a single or unitary component. The proximal end of the ultrasonic surgical instrument 100 receives and is fitted to the distal end of the ultrasonic transducer 50 by insertion of the transducer into the handle assembly 68. The ultrasonic surgical instrument 100 may be attached to and removed from the ultrasonic transducer 50 as a unit. The ultrasonic surgical instrument 100 may include a handle assembly 68, comprising mating housing portions 69 and 70 and an ultrasonic transmission assembly 71. The elongated transmission assembly 71 of the ultrasonic surgical instrument 100 extends orthogonally from the instrument handle assembly 68.

The handle assembly 68 may be constructed from a durable plastic, such as polycarbonate or a liquid crystal polymer. It is also contemplated that the handle assembly 68 may alternatively be made from a variety of materials including other plastics, ceramics or metals.

The transmission assembly 71 includes a waveguide 80 and a blade 79. It will be noted that, in some applications, the transmission assembly is sometimes referred to as a "blade assembly". The waveguide 80, which is adapted to transmit ultrasonic energy from transducer 50 to the tip of blade 79 may be flexible, semi-flexible or rigid. The waveguide 80 may also be configured to amplify the mechanical vibrations transmitted through the waveguide 80 to the blade 79 as is well known in the art. The waveguide 80 may further have features to control the gain of the longitudinal vibration along the waveguide 80 and features to tune the waveguide 80 to the resonant frequency of the system. In particular, waveguide 80 may have any suitable cross-sectional dimension. For example, the waveguide 80 may have a substantially uniform cross-section or the waveguide 80 may be tapered at various sections or may be tapered along its entire length. Ultrasonic waveguide 80 may, for example, have a length substantially equal to an integral number of one-half system wavelengths (nλ/2). The ultrasonic waveguide 80 and blade 79 may be preferably fabricated from a solid core shaft constructed out of material, which propagates ultrasonic energy efficiently, such as titanium alloy (i.e., Ti-6AI-4V), aluminum alloys, sapphire, stainless steel or any other acoustically compatible material.

Ultrasonic waveguide 80 may further include at least one radial hole or aperture 66 extending therethrough, substantially perpendicular to the longitudinal axis of the waveguide 80. The aperture 66, which may be positioned at a node, is configured to receive a connector pin 27, discussed below, which connects the waveguide 80, to the outer sheath 72. Proximal o-ring 67a and distal o-ring 67b are assembled onto transmission assembly 71 near the nodes.

Blade 79 may be integral with the waveguide 80 and formed as a single unit. In an alternate expression of the current embodiment, blade 79 may be connected by a threaded connection, a welded joint, or other coupling mechanisms. The distal end of blade 79, or blade tip 79a, is disposed near an anti-node in order to tune the acoustic assembly to a preferred resonant frequency fₒ when the acoustic assembly is not loaded by tissue. When ultrasonic transducer 50 is energized the blade tip 79a is configured to move substantially longitudinally (along the x axis) in the range of, for example, approximately 10 to 500 microns peak-to-peak, and preferably in the range of about 20 to about 200 microns at a predetermined vibrational frequency fₒ of, for example, 55,500 Hz. Blade tip 79a also preferably vibrates in the y-axis at about 1 to about 10 percent of the motion in the x-axis.

One embodiment of waveguide 80 and blade 79 is product code HF105 sold by Ethicon Endo-Surgery, Inc. of Cincinnati, Ohio and further disclosed in U.S. Patent No. 6,423,082, entitled ULTRASONIC SURGICAL BLADE WITH IMPROVED CUTTING AND COAGULATION FEATURES. Other blade designs are also contemplated for use with the current invention, including product code DH105 sold by Ethicon Endo-Surgery, Inc. and further disclosed in U.S. Patent No. 5,324,299, entitled ULTRASONIC SCALPEL BLADE AND METHODS OF APPLICATION. Other ultrasonic blade designs are also useful as is well known to those skilled in the art.

Waveguide 80 is positioned within outer sheath 72 and held in place via pin 27. Preferably pin 27 is made of any compatible metal, such as stainless steel or titanium or a durable plastic, such as polycarbonate or a liquid crystal polymer. In a first expression of one embodiment, pin 27 is partially coated with an elasto-meric material, such as silicon for that portion 29 of pin 27 that extends through waveguide 80. The silicone provides insulation from the vibrating blade throughout the length of hole 66. This enables high efficiency operation whereby minimal overheating is generated and maximum ultrasonic output power is available at the blade tip for cutting and coagulation.

Outer sheath 72 passes through an aperture 210 of release button 200. Positioned below release button and within housing portion 69 is a spring 220 that asserts an upward force on release button 200. The upward force causes aperture 210 to firmly assert pressure against outer sheath 72 and thereby prevents outer sheath 72 and waveguide 80 and blade 79 from either rotating within handle 68 or axially translating with respect to handle 68. When the user exerts a downward force on release button 200, the spring is compressed and it no longer asserts a holding force on outer sheath 72. The user may then axially translate outer sheath 72 and waveguide 80 and blade 72 relative to handle 68 and/or rotate the outer sheath and waveguide 80 and blade 72 relative to handle 68.

Housing 68 includes a proximal end, a distal end, and a cavity 59 extending longitudinally therein. Cavity 59 is configured to accept a switch assembly 300 and the transducer assembly 50. In one expression of the current embodiment, the distal end of transducer 50 threadedly attaches to the proximal end of transmission rod 80. The distal end of transducer 50 also interfaces with switch assembly 300 to provide the surgeon with finger-activated controls on surgical instrument 19.

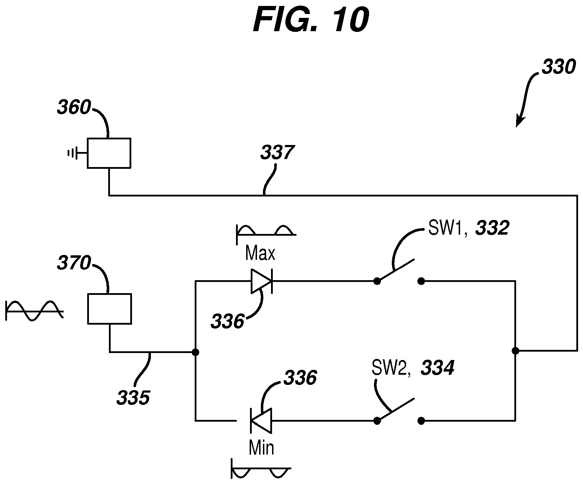

Transducer 50 includes a first conductive ring 400 and a second conductive ring 410 which are securely disposed within the transducer body 50 as is described in co-pending application serial no. 11/545,784. Switch assembly 300 comprises a pushbutton assembly 310, a circuit assembly 330, a switch housing 350, a first pin conductor 360 and a second pin conductor 370 (see FIG 10). Switch housing 350 is annular-shaped and is supported within handle assembly 68 by way of corresponding supporting mounts on switch housing 350 and housing portions 69 and 70.

With reference also to FIG. 10, pins 360 and 370 are electrically connected to dome switches 332 and 334 via conductors 337 and 335, respectively, at one end and to the distal end of transducer 50 at a second end. Pins 360 and 370 each have spring-loaded tips that interface with transducer 50. Each end spring-loaded tip has a 0.050-inch working travel to allow for manufacturing tolerances associated with the stack up of the assembled parts.

A circuit 330 provides for the electro-mechanical interface between pushbuttons 321 and 322 and the generator 30 via transducer 50. Circuit 330 comprises two dome switches 332 and 334 that are mechanically actuated by depressing pushbuttons 321 or 322, respectively. Dome switches 332 and 334 are electrical contact switches, that when depressed provide an electrical signal to generator 30 as shown by the electrical wiring schematic of Fig. 10. Circuit 330 also comprises two diodes within a diode package 336 and conductors, 335 and 337 as is known to those in the art, that connect to pins 360 and 370, respectively, which in turn provide electrical contact to ring conductors 400 and 410 (not shown), which in turn are connected to conductors in cable 22 that connect to generator 30.

As is readily apparent, by depressing pushbuttons 321 and 322 the corresponding contact surfaces depress against corresponding dome switches 332 and 334 to activate the circuit illustrated in FIG. 10. When the surgeon depresses 321 pushbutton, the generator will respond with a certain energy level, such as a maximum ("max") power setting; when the surgeon depresses pushbutton 322, the generator will respond with a certain energy level, such as a minimum ("min") power setting, which conforms to accepted industry practice for pushbutton location and the corresponding power setting.

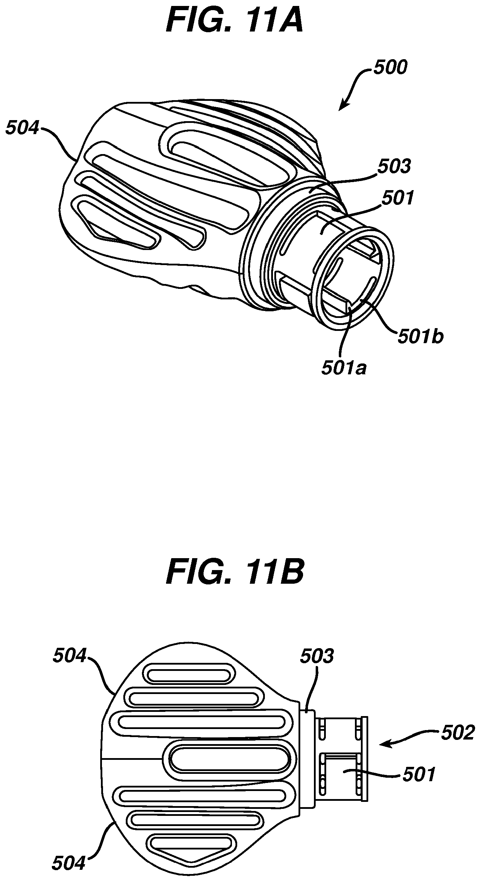

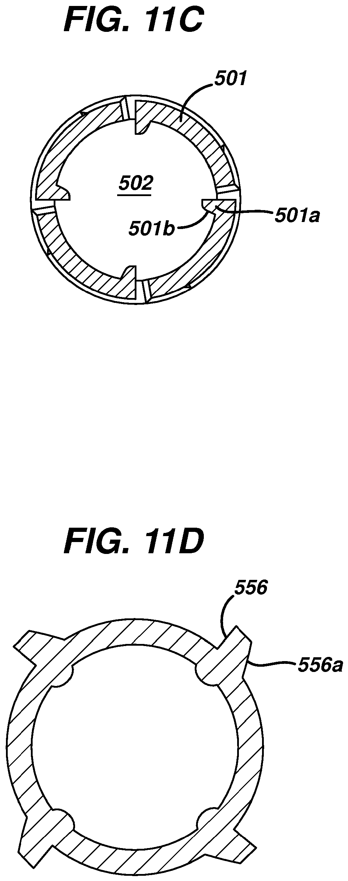

Referring also now to Figs. 11A-D, a two-piece torque wrench 450 is shown. The torque wrench includes a hand wrench 500 and an adaptor 550. In one embodiment, hand wrench 500 is provided with cantilever arms 501 disposed in an annular fashion about the centerline of hand wrench 500. Cantilever arms 501 include teeth 501a disposed, in one embodiment, in an inward perpendicular fashion in relation to cantilever arms 501. Teeth 501a, in one embodiment of the current invention, are disposed with a cam ramp 501b at a 25° angle with respect to the perpendicular angle between arm 501 and teeth 501a. Lumen 502 extends the entire length of hand wrench 500 for accepting adaptor 550.

Adaptor 550 has a longitudinal shaft 552 with cantilevered tabs 554 at its distal end. At the proximal end of shaft 552 are spline gears 556 projecting in a perpendicular fashion along the outer circumference of shaft 552. Spline gears 556 include cam ramps 556a disposed at an angle from about 23° to about 28° with respect to the perpendicular angle between the outer circumference of shaft 552 and spline gears 556. Adaptor further includes an interface 560 rigidly connected to shaft 552 and defining an opening for rigidly engaging the distal end of outer sheath 72.

In assembly, torque wrench opening 502 is aligned with shaft 552 and guided along substantially the entire length of shaft 552 until the tabs 554 flex inward and capture shoulder 505 at the distal end of hand wrench 500. Cam ramp 501b slidably engages retainer cam ramps 556a. The torque wrench assembly 450 slidably engages the distal end of outer sheath 72 and is held rigidly in place. Flat surfaces of interface 560 mate with flat surfaces (not shown) at the distal end of outer sheath 72.

Clockwise annular motion or torque is imparted to hand wrench 500 through paddles 504. The torque is transmitted through arms 501 and teeth 501a to gears 556, which in turn transmit the torque to the waveguide 80 via outer shroud 72 via insulated pin 27. When a user imparts 5-12 lbs. of torque, the ramps 501b and 556 cause the arms 501 to move or flex away from the centerline of wrench 500 ensuring that the user does not over-tighten the waveguide 80 onto transducer 50. When a counter-clockwise torque is applied to wrench 500 via paddles 504, the perpendicular flat sides of teeth 501a and 556 abut allowing a user to impart a torque to the interface between the waveguide 80 and transducer 50 in proportion to the force applied to the paddles facilitating removal of the instrument 100 from the transducer 50. The torque wrench 450 may be constructed from a durable plastic, such as polycarbonate or a liquid crystal polymer. It is also contemplated that the wrench 450 may alternatively be made from a variety of materials including other plastics, ceramics or metals.

In another embodiment (not shown), the paddles and cantilever arm assembly may be separate components attached by mechanical means or chemical means such as adhesives or glue.

Preferably, the ultrasonic apparatus 100 described above will be processed before surgery. First, a new or used ultrasonic apparatus 100 is obtained and if necessary cleaned. The ultrasonic apparatus can then be sterilized. In one sterilization technique the ultrasonic apparatus is placed in a closed and sealed container, such as a plastic or TYVEK bag. Optionally, the ultrasonic apparatus can be combined in the container as a kit with other components, including a torque wrench 450. The container and ultrasonic apparatus, as well as any other components, are then placed in a field of radiation that can penetrate the container, such as gamma radiation, x-rays, or high-energy electrons. The radiation kills bacteria on the ultrasonic apparatus and in the container. The sterilized ultrasonic apparatus can then be stored in the sterile container. The sealed container keeps the ultrasonic apparatus sterile until it is opened in the medical facility.

### Pushbutton Activation

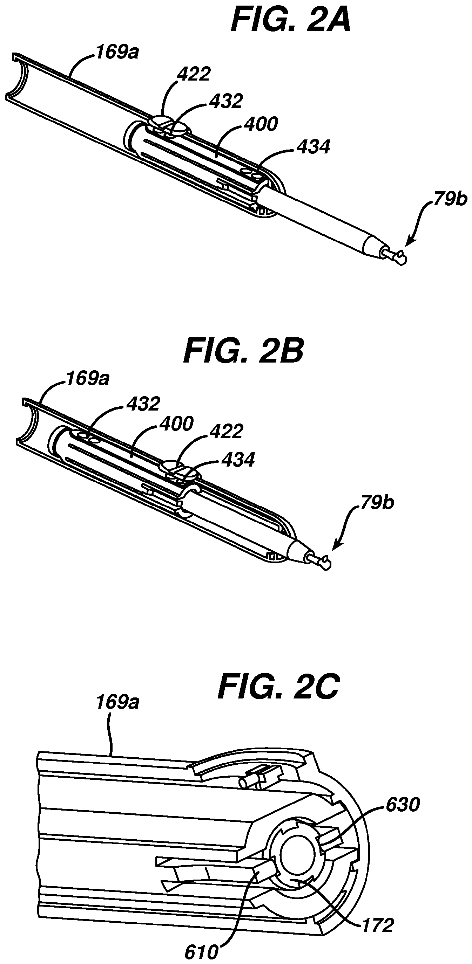

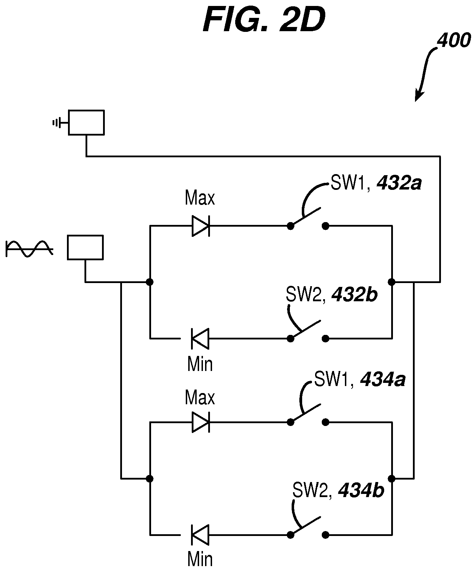

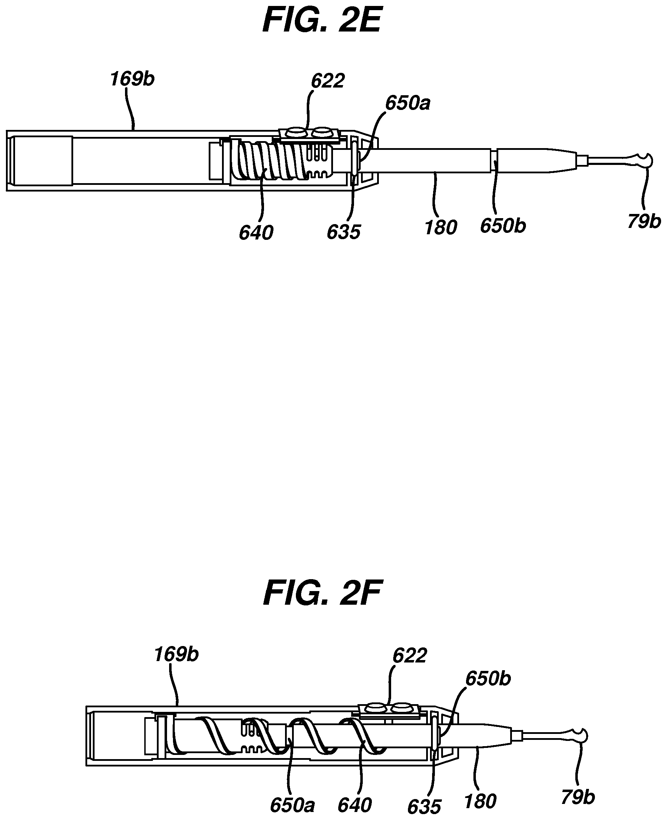

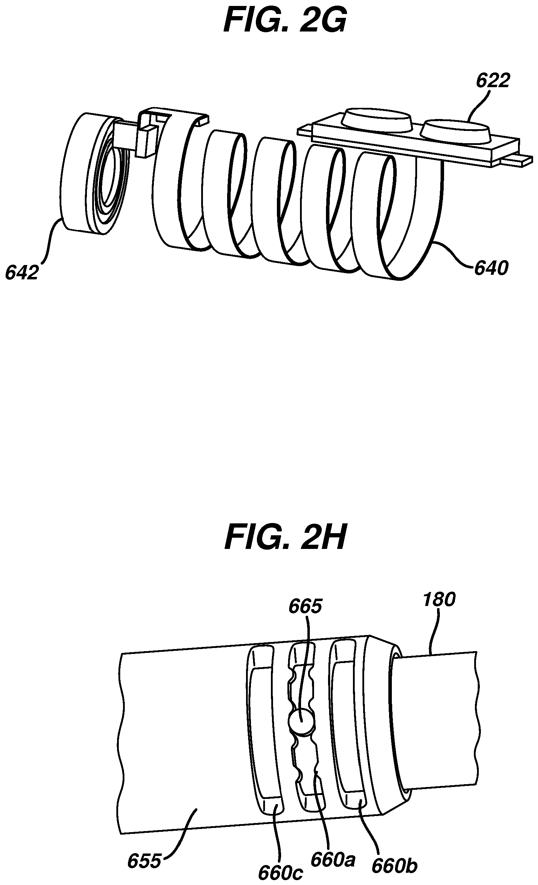

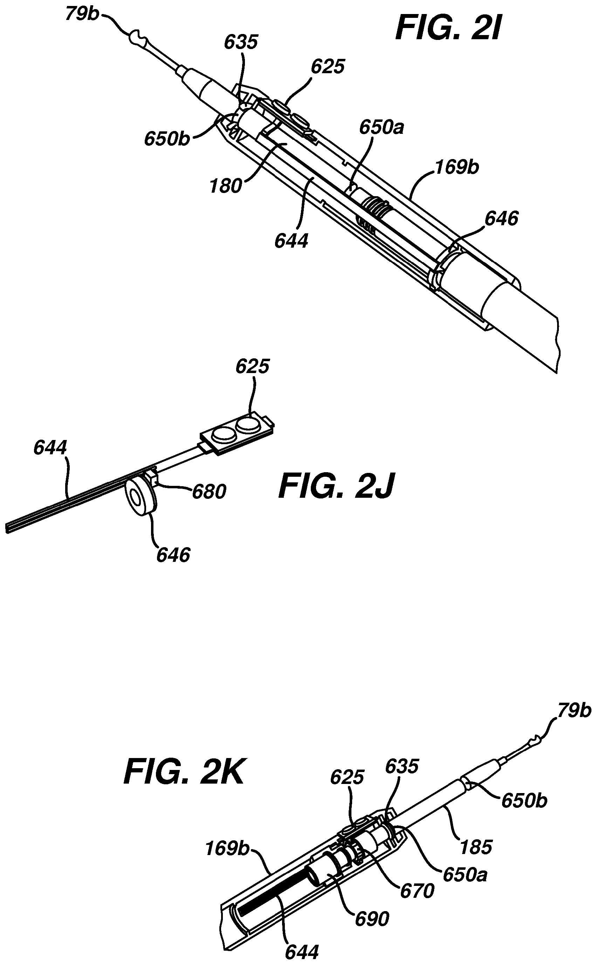

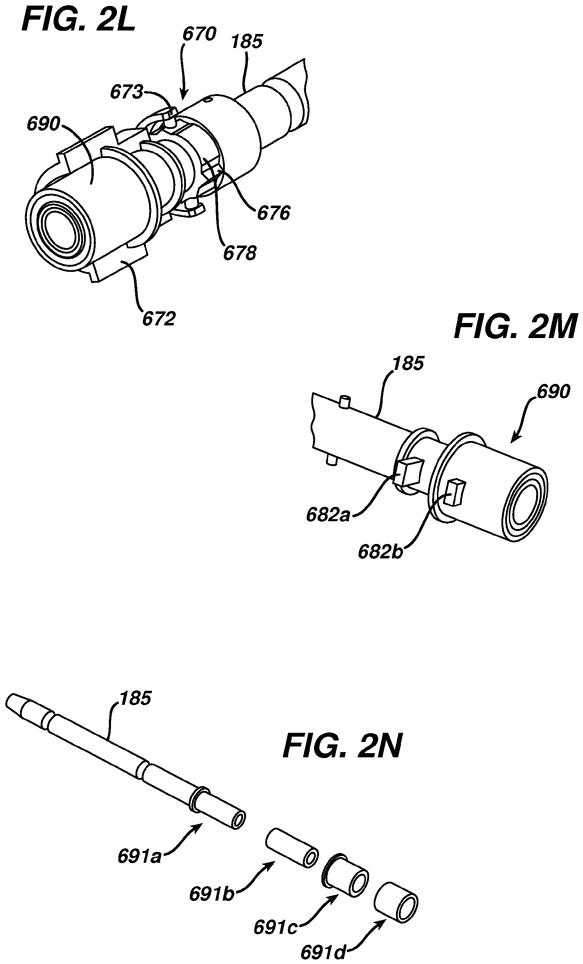

FIGS. 2A-B illustrate an alternate expression for the electrical connection between the activation buttons and the handpiece. Activation button 422 is stationary on housing 169a. Blade and sheath assembly 400 contains 2 separate dome switch locations, which each contain two dome switches, 432a, 432b, and 434a, 434b. Blade assembly 400 translates to allow for rocker button 422 to activate the device through proximal dome switches 432 or distal dome switches 432. When dome switches 432 are in use, end effector 79b is extended relative to housing 169a. When dome switches 434 are used, end effector 79b is retracted relative to device housing 169a. The electrical schematic of FIG. 2D illustrates both sets of switches in parallel electrical connection.

Referring to FIGS. 2E-G, an alternate expression of the electrical connection between the handpiece 50 and activation switches utilizes a spiral flex circuit 640. Spiral flex circuit 640 carries the connection wires between the switches and handpiece 50 of FIG. 2D. Dome switches 622 are connected to hand piece connector 642 through spiral flex circuit 640. Spiral flex circuit 640 allows sheath 180 to translate with respect to housing 169b while dome switches and button 622 remain stationary on housing 169b. Dome switches 622 maintain a connection with transducer 50 at all times via spiral flex circuit 640.

Another expression for the electrical connection is found in rail design 644 of FIGS. 2I-K. Brushes 680 are used to maintain an electrical connection between activation button 625 and hand piece connector 646 through electric rail 644. Rail 644 maintains the connection as sheath 180 is translated between distal position 650b and proximal position 650a.

### Axial Translation

Referring now to Figures 2E-F, a means for translation exists on sheath 180. Detent features 650a and 650b are spaced apart on blade sheath 180 and create two positions for end effector 79b relative to the housing 169b. Blade sheath 180 may also contain additional detent features, which would create additional positions for end effector 79b. Detent clip 635 locks shaft 180 into proximal detent position 650a or distal detent position 650b. User force disengages detent clip 635 and allows shaft 180 to translate from locking position 650a to 650b or 650b to 650a. These detent features are utilized for translation of end effector 79b with respect to housing 169b.

### Rotation

Referring to FIG. 2C, to allow for rotation of the end effector relative to housing 169a, blade sheath 172 contains 4 equally spaced detent features 630 that capture fingers 610. Fingers 610 move in and out of detent features 630. This allows for end effector 79b to rotate and lock in 4 different positions, 90 degrees apart. An alternate expression for rotation is shown at FIG. 2H. Pin 665 attached to sheath 180 allows for rotation in detent feature 660a. Detent feature 660a is composed of 5 features spaced equally apart in connector 655. Slots 660b and 660c on either side of 660a allow for compliance to provide the desired detent torque to rotate end effector 79b with respect to housing 169b.

Shown at FIG. 2L, detent wheel assembly 670 is used to allow 360-degree rotation of end effector 79b and using the rail feature of FIGS 2I-K. A non-slidable connector 672 is rotatably fixed relative to rails 644. Positioned on connector 672 are tabs 673 spaced 180 degrees apart and contact detent wheel 676 positioned over sheath 185. Detent teeth 678 are equally spaced around detent wheel 676. As end effector 79b is rotated with respect to housing 169c, tabs 673 move in and out of teeth 678 locking end effector 79b in place. This allows the user to change the angle with which the end effector is used with respect to housing 169c. Positioned within openings of connector 672 (not shown) are two electrical bushings 682a and 682b, which contact stationary rail 644 and concentric cylinder assembly 690. Cylinder assembly 690 comprises a proximal cylindrical connection 691a on sheath 185. An electrical cylindrical element 691b concentrically fits around connection 691a, and a cylindrical insulator 691c concentrically fits around electrical element 691b, and electrical cylindrical element 691d fits around insulator 691c. Electrical bushings 682a and 682b contact cylindrical elements 691b and 691d, respectively on one connection point and rail 644 at an opposite connection point. Electrical bushings 682a and 682b provide electrical connection to handpiece 50, thus providing a continuous electrical connection between pushbuttons 625 and handpiece 50.

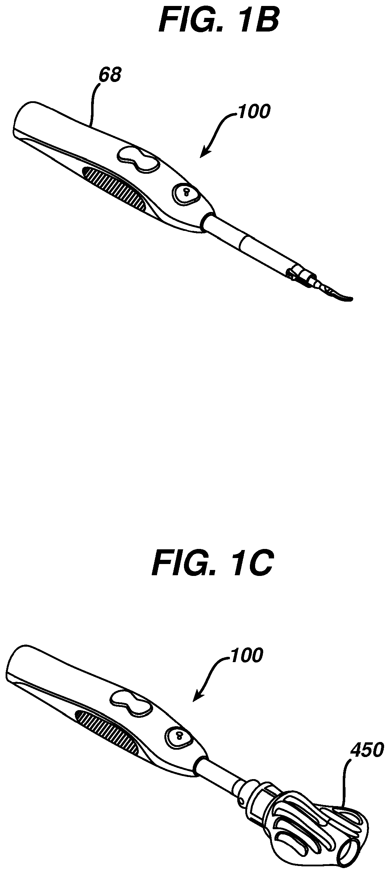

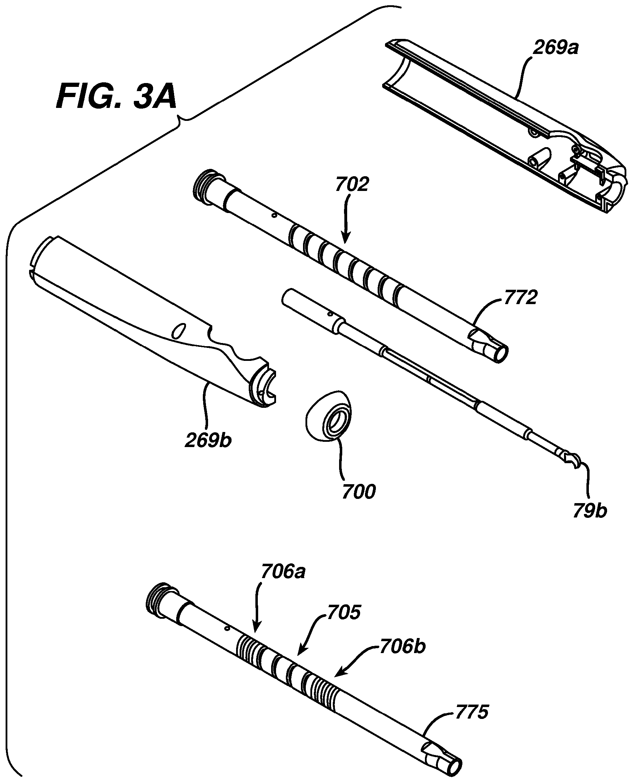

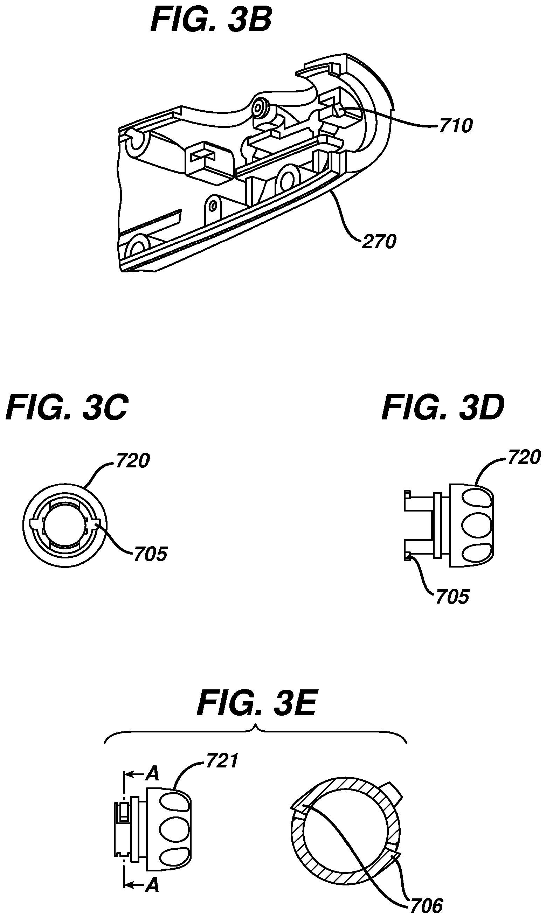

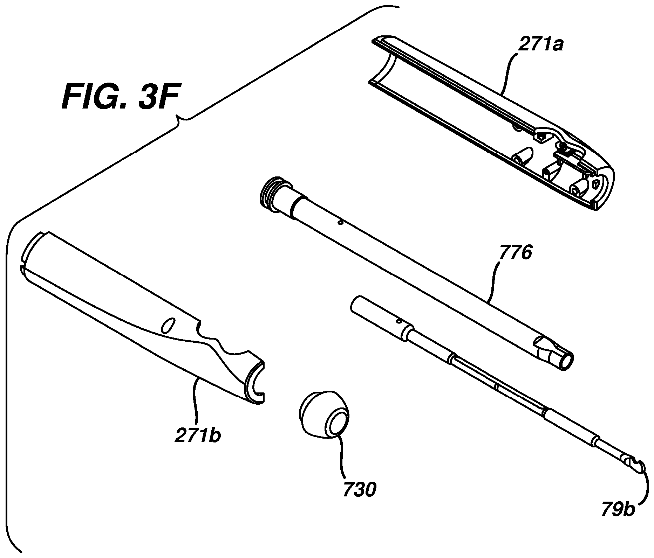

Referring now to FIGS. 3A-F and FIG. 1, alternate embodiments for telescoping and rotating end effector 79b are shown. Blade sheath 772 contains grooves 702 in the form of a helix. Grooves 702 interact with end cap 700 in the same fashion as a nut and bolt. Sheath 772, grooves 702 and end cap 700 are all contained within housing halves 269a and 269b. As sheath 772 is rotated, end effector 79b will advance and retract with respect to housing 269. Rotation and translation happen simultaneously and are not independent of each other. An alternate expression of the current embodiment is found in sheath 775. Sheath 775 contains grooves 705, which has a change in pitch near proximal and distal ends 706a and 706b respectively. This change in pitch allows for further precision near maximum and minimum extension of end effector 79b relative to housing 269. While rotation and translation are still dependent on each other, grooves 705 gives the user more positions for end effector 79b relative to housing 269.

Reference is now made to FIGS. 3B-F illustrating additional embodiment for a friction lock mechanism. Knob 720 contains teeth 705, which are equally spaced 180 degrees apart. Knob 720 fits into the distal end of housing 270. Slanted surface 710 within housing 270 acts as a ramp and interferes with flexing teeth 705 inward when knob 720 is rotated. This interference creates compressive forces and locks the blade sheath in place with respect to housing 270. When the interference is relieved, the blade sheath is free to translate and rotate using any of the previous discussed translation and rotation embodiments. An alternate expression of knob 720 is knob 721. Knob 721 contains teeth 706 which are cut out and hinged on knob 721. As knob 721 is rotated in housing 270, teeth 706 flex inward and create compressive forces to lock the blade sheath with respect to the sheath 270.

An alternate expression to the friction lock is knob 730. When knob 730 is rotated it creates interference between housing 271a and 271b. This interference causes knob 730 to deflect, and applies compressive forces and friction to sheath 776 locking it in place. When knob 730 is not creating interference, end effector 79b is able to translate and rotate with respect to housing 271.

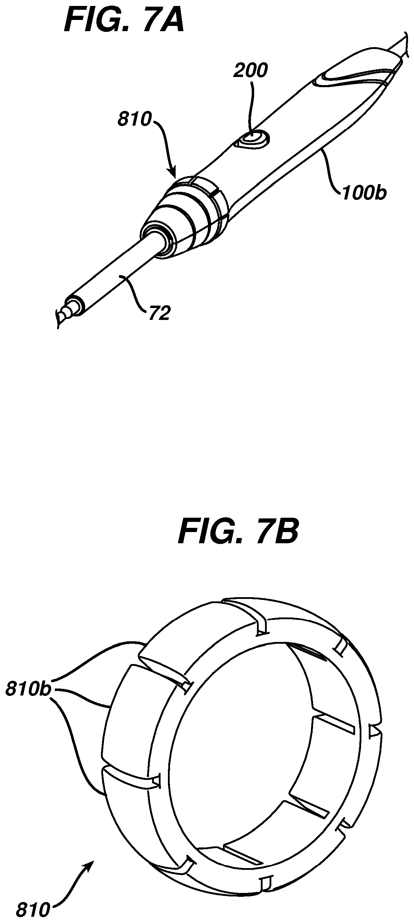

FIG. 7A and 7B illustrate an alternative embodiment for activation buttons. Ultrasonic instrument 100b contains ring activation button assembly 810. Ring activation button assembly 810 contains eight segments 810b. A minimum of two button segments 810b must be contacted to activate harmonic energy in ultrasonic instrument 100b. Ultrasonic instrument 100b can be configured to accommodate different user grips. Instrument 100b can activate upon user contact with a second ring segment 810b, or after contact with a third ring segment 810b depending on how many of the user's fingers are used to hold the device. Ring activation button assembly 810 would allow ultrasonic instrument 100b to be easily rotated in a surgeon's hand while maintaining harmonic energy to the targeted area.

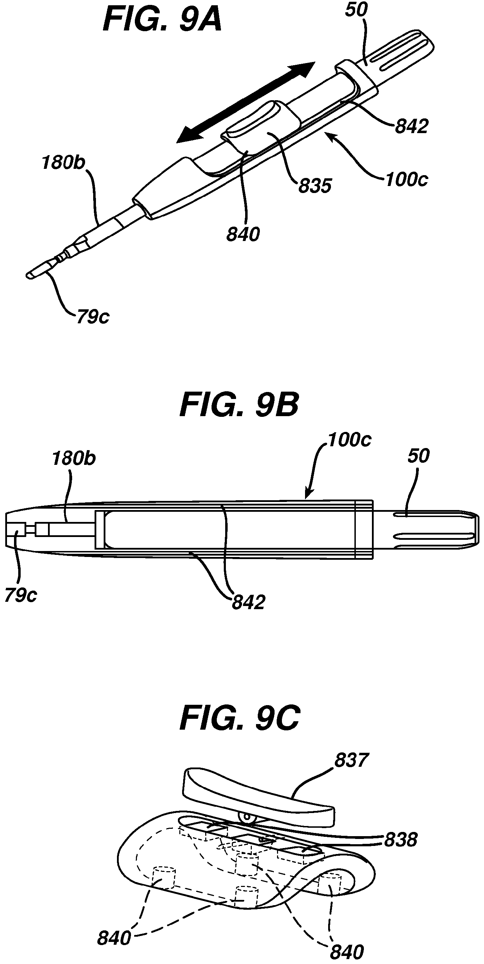

**.** Referring now to Figure 9A-C, in an alternate embodiment, a removable activation button assembly 835 translates longitudinally along metal rails 842 carried by ultrasonic surgical instrument 100c. Magnetic rails are electrically connected to the handpiece or transducer 50 as would be readily apparent to a skilled artesian. Activation button assembly 835 contains one or more magnets 840 to anchor onto and form an electrical connection with metal rails 842. Magnet 840 is covered in an electrically conductive material and wired to dome switches 838. Dome switches are activated by, for example, a rocker switch 837; however any type of switch is available as is known to the skilled artesian. Activation button assembly 835 can be rotated on ultrasonic surgical instrument 100c to switch the location of max and min rocker button 837. Activation button assembly 835 can also move in a sliding fashion to any place on ultrasonic surgical instrument 100c where magnet 840 holds assembly 835 in place on metal rails 842. This allows for a variable distance between end effector 79c and activation assembly 835. In one expression of this embodiment, end effector 79c and shaft 180b are fixed relative to housing 68, as shown in FIG. 9A; alternatively, end effector 79c and shaft 180b are able to move relative to housing 68, shown in FIG. 9B (end effector in proximal position).

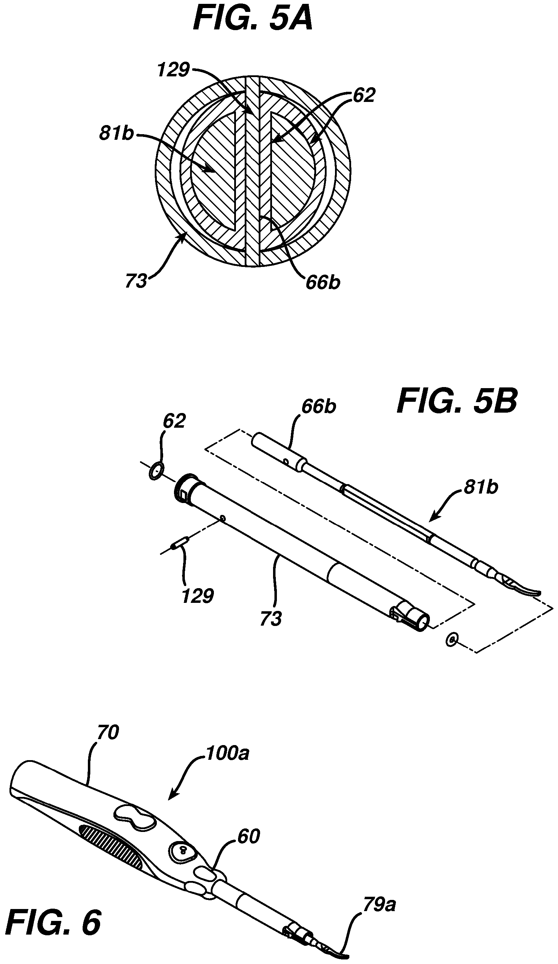

An alternate expression for alignment pin 27 is found in FIGS. 5A and 5B. Hole 66b and rear bumper 62 are relocated to the place of minimum displacement on blade 81b. Rear bumper 62 is over molded onto blade 81b with an elasto-meric material such as silicon. The inside walls of through hole 66b are also insert molded with an elasto-meric material such as silicone. Alignment pin 129 is no longer coated with a material, and is pressed through blade sheath 73, rear bumper 62, and blade 81b. This process would be a cost savings on the alignment pin related to the elimination of the secondary insert-molding step of the alignment pin. There would also be an acoustical improvement and a slight heat reduction as over molded rear bumper 62 and pin 129 could both be placed at the location of minimum displacement.

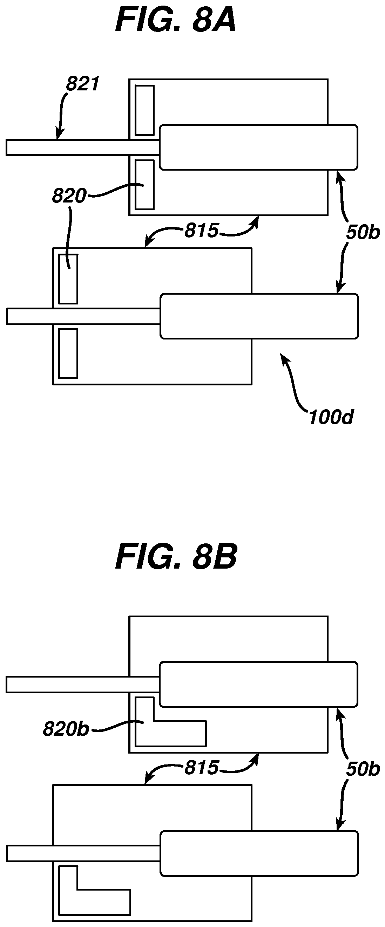

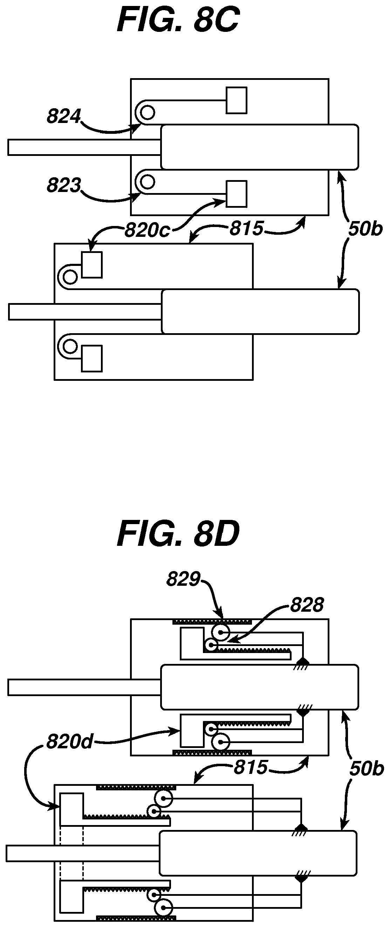

Referring now to FIG. 8A-D, an additional embodiment for the ultrasonic instrument 100 is counterbalance feature 820. In a first embodiment counterbalance feature 820 remains in a fixed position inside housing 815 and provides a statically balances instrument 100 with respect to the multiple positions of handpiece 50b relative to housing 815. An alternate expression utilizes counterbalance 820b in the form of an annulus or asymmetric shape.

A further expression for counterbalance system is one that dynamically balances instrument 100 with respect to he multiple positions of handpiece 50b with respect to housing 815. Counterbalance 820c is moved inside housing 815 by band 823 and post 824. Band 823 is grounded to handpiece 50b. As handpiece 50b retracts proximally, counterbalance 820c is moves distally through the pulling of handpiece 50b on band 823 around post 824. Once adjusted, counterbalance 820c is located further from handpiece 50b to better balance ultrasonic instrument 100c. As handpiece 50b is extends distally, counterbalance 820c moves proximally toward the center of mass of the system.

Counterbalance 820d of FIG. 8D is an alternate expression for the movable counterbalance mechanism. As hand piece 50b retracts proximally and distally, counterbalance 820d is shifted via pinion gears 828, and rack gear 829. Pinion gears 828 are grounded to handpiece 50b. Pinion gears 828 could also be grounded to other ultrasonic surgical instrument 100c components. This movement of counterbalance 820d will counteract the weight of handpiece 50b. The counterbalance system would give optimal balance of the device for all positions of the end effector relative to the activation buttons. This would give the user better precision when operating the device.

Referring now to FIG. 6 and FIG. 1A, an alternate embodiment for ultrasonic surgical instrument 100a includes LED ring 60. LED ring 60 is composed of one or more LED or other low power light sources spaced evenly apart on the distal end of housing 70. One skilled in the art may determine an alternate light source or configuration to achieve the objective of pointing light in the direction of harmonic blade end effector 79a. LED ring 60 may be powered by an external power source or battery pack. LED ring 60 may be activated with buttons 332 and 334 that also activate generator 300. LED ring 60 may also be continuously on or activated through a separate switch.

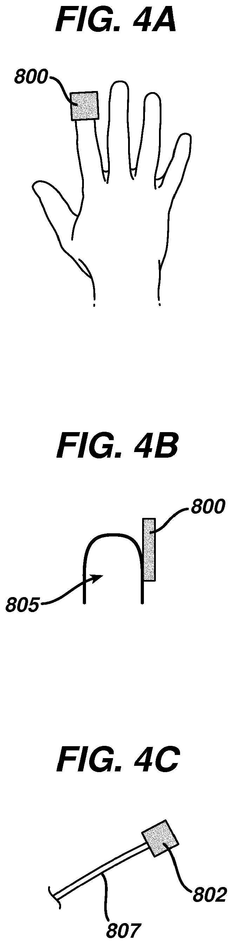

Part of a kit to go along with the device could include a means to better coagulate vessels. Referring now to FIG. 4A-C, hand held tissue pad 800 consists of Teflon or another compatible material chosen by one skilled in the art to interact with the harmonic blade. Pad 800 is attached to finger hole 805 and resides on the surgeon's non-dominant hand. Pad 800 is used to apply pressure to blood vessels against blade end effector 79a which closes the vessels. An alternate expression consists of pad 802 on stick 807. Stick 807 is held in the surgeon's non-dominant hand and is used to apply pressure to blood vessels against blade end effector 79a which closes the vessels. This allows for improved hemostasis and blade multifunctionality.

While the present invention has been illustrated by description of several embodiments, it is not the intention of the applicant to restrict or limit the scope of the invention to such detail. Numerous variations, changes, and substitutions will occur to those skilled in the art without departing from the scope of the invention. Accordingly, it is intended that the invention be limited only by the scope of the appended claims.

## Claims

1. An ultrasonic surgical instrument (100) comprising a housing (68) defining a longitudinal axis, a power activation element (422) on the housing and an end effector (79b), the power activation element and the end effector configured to be separated by a first distance, at which the end effector (79b) is effective to treat tissue and the power activation element (422) is operable to activate the end effector, and
**characterized in that** the ultrasonic surgical instrument further comprises a translating element for separating the power activation element and the end effector by a second distance, at which the end effector is effective to treat tissue and the power activation element is operable to activate the end effector, wherein the end effector or the power activation element translates with respect to the longitudinal axis.

2. The ultrasonic surgical instrument of claim 1, wherein the power activation element is stationary.

3. The ultrasonic surgical instrument of claim 1, wherein the end effector is stationary.

4. The ultrasonic surgical instrument of claim 1, wherein the end effector rotates relative to the power activation element.

5. The ultrasonic surgical instrument of claim 1, wherein:
the power activation element is defined by a housing assembly that also defines a longitudinal axis;
the translating element comprises an outer tube (72) slidably supported by and extending distally from the housing assembly and having a proximal end and a distal end;
an ultrasonic waveguide (80) having a proximal end and a distal end is positioned within the outer tube; and
the end effector comprises an ultrasonically actuated blade (79a) positioned to the distal end of the waveguide.

6. The ultrasonic surgical instrument of claim 5 further comprising a locking element supported by the housing assembly, wherein the locking element (635) provides a stop to prevent the outer tube from translating relative to the handle assembly.

7. The ultrasonic surgical instrument of claim 6, wherein the locking element rotates with respect to the handle assembly.

8. The ultrasonic surgical instrument of claim 6, wherein the locking element moves in a direction normal to the longitudinal axis.

9. The ultrasonic surgical instrument of claim 5, wherein the handle assembly rotatably supports the outer tube.

10. The ultrasonic surgical instrument of claim 9, wherein the outer tube comprises grooves (702).

11. The ultrasonic surgical instrument of claim 5 further comprising an illumination assembly.

12. The ultrasonic surgical instrument of claim 5 further comprising a counterbalance assembly (820c), wherein the counterbalance assembly provides a weight to offset the movement of the outer tube.

13. The ultrasonic surgical instrument of claim 1, wherein:
the power activation element comprises an actuator assembly slidably supported by a handle assembly that also defines a longitudinal axis;
an outer tube is supported by and extends distally from the handle assembly and has a proximal end and a distal end;
an ultrasonic waveguide having a proximal end and a distal end is positioned within the outer tube; and
the end effector comprises an ultrasonically actuated blade positioned to the distal end of the waveguide.

14. The ultrasonic surgical instrument of claim 13, wherein the handle assembly slidably supports the outer tube.

## Patentansprüche

1. Chirurgisches Ultraschallinstrument (100), umfassend ein Gehäuse (68), das eine Längsachse definiert, ein Stromaktivierungselement (422) an dem Gehäuse und einen Endeffektor (79b), wobei das Stromaktivierungselement und der Endeffektor dazu ausgelegt sind, durch einen ersten Abstand voneinander getrennt zu sein, bei dem der Endeffektor (79b) wirksam ist, um Gewebe zu behandeln, und das Stromaktivierungselement (422) bedienbar ist, um den Endeffektor zu aktivieren, und
**dadurch gekennzeichnet, dass** das chirurgische Ultraschallinstrument ferner ein Translationselement zum Trennen des Stromaktivierungselements und des Endeffektors durch einen zweiten Abstand umfasst, bei dem der Endeffektor wirksam ist, um Gewebe zu behandeln, und das Stromaktivierungselement bedienbar ist, um den Endeffektor zu aktivieren, wobei sich der Endeffektor oder das Stromaktivierungselement mit Bezug auf die Längsachse bewegt.

2. Chirurgisches Ultraschallinstrument nach Anspruch 1, wobei das Stromaktivierungselement ortsfest ist.

3. Chirurgisches Ultraschallinstrument nach Anspruch 1, wobei der Endeffektor ortsfest ist.

4. Chirurgisches Ultraschallinstrument nach Anspruch 1, wobei sich der Endeffektor bezogen auf das Stromaktivierungselement dreht.

5. Chirurgisches Ultraschallinstrument nach Anspruch 1, wobei:
das Stromaktivierungselement von einer Gehäuseanordnung definiert wird, die ebenfalls eine Längsachse definiert;
das Translationselement eine äußere Röhre (72) umfasst, die verschiebbar an der Gehäuseanordnung gelagert ist und sich distal davon erstreckt und ein proximales und ein distales Ende aufweist;
ein Ultraschallwellenleiter (80), der ein proximales Ende und ein distales Ende aufweist, im Innern der äußeren Röhre positioniert ist; und
der Endeffektor eine mit Ultraschall betätigte Klinge (79a) umfasst, die am distalen Ende des Wellenleiters positioniert ist.

6. Chirurgisches Ultraschallinstrument nach Anspruch 5, ferner umfassend ein Verriegelungselement, das an der Gehäuseanordnung gelagert ist, wobei das Verriegelungselement (635) einen Anschlag bereitstellt, um zu verhindern, dass sich die äußere Röhre bezogen auf die Griffanordnung bewegt.

7. Chirurgisches Ultraschallinstrument nach Anspruch 6, wobei sich das Verriegelungselement bezogen auf die Griffanordnung dreht.

8. Chirurgisches Ultraschallinstrument nach Anspruch 6, wobei sich das Verriegelungselement in eine Richtung senkrecht zur Längsachse bewegt.

9. Chirurgisches Ultraschallinstrument nach Anspruch 5, wobei die Griffanordnung die äußere Röhre drehbar lagert.

10. Chirurgisches Ultraschallinstrument nach Anspruch 9, wobei die äußere Röhre Nuten (702) umfasst.

11. Chirurgisches Ultraschallinstrument nach Anspruch 5, ferner umfassend eine Beleuchtungsanordnung.

12. Chirurgisches Ultraschallinstrument nach Anspruch 5, ferner umfassend eine Ausgleichsanordnung (820c), wobei die Ausgleichsanordnung ein Gewicht bereitstellt, um die Bewegung der äußeren Röhre auszugleichen.

13. Chirurgisches Ultraschallinstrument nach Anspruch 1, wobei:
das Stromaktivierungselement eine Aktoranordnung umfasst, die verschiebbar an einer Griffanordnung gelagert ist, die ebenfalls eine Längsachse definiert;
eine äußere Röhre an der Griffanordnung gelagert ist und sich distal davon erstreckt und ein proximales und ein distales Ende aufweist;
ein Ultraschallwellenleiter, der ein proximales Ende und ein distales Ende aufweist, im Innern der äußeren Röhre positioniert ist; und
der Endeffektor eine mit Ultraschall betätigte Klinge umfasst, die an dem distalen Ende des Wellenleiters positioniert ist.

14. Chirurgisches Ultraschallinstrument nach Anspruch 13, wobei die Griffanordnung die äußere Röhre verschiebbar lagert.

## Revendications

1. Instrument chirurgical à ultrasons (100) comprenant un logement (68) définissant un axe longitudinal, un élément d'activation de puissance (422) sur le logement et un effecteur terminal (79b), l'élément d'activation de puissance et l'effecteur terminal étant configurés pour être séparés par une première distance, à laquelle l'effecteur terminal (79b) est efficace pour traiter un tissu et l'élément d'activation de puissance (422) est exploitable pour activer l'effecteur terminal, et
**caractérisé en ce que** l'instrument chirurgical à ultrasons comprend en outre un élément de translation pour séparer l'élément d'activation de puissance et l'effecteur terminal par une seconde distance, à laquelle l'effecteur terminal est efficace pour traiter un tissu et l'élément d'activation de puissance est exploitable pour activer l'effecteur terminal, dans lequel l'effecteur terminal ou l'élément d'activation de puissance se déplace par translation par rapport à l'axe longitudinal.

2. Instrument chirurgical à ultrasons selon la revendication 1, dans lequel l'élément d'activation de puissance est immobile.

3. Instrument chirurgical à ultrasons selon la revendication 1, dans lequel l'effecteur terminal est immobile.

4. Instrument chirurgical à ultrasons selon la revendication 1, dans lequel l'effecteur terminal tourne par rapport à l'élément d'activation de puissance.

5. Instrument chirurgical à ultrasons selon la revendication 1, dans lequel :
l'élément d'activation de puissance est défini par un ensemble logement qui définit également un axe longitudinal ;
l'élément de translation comprend un tube externe (72) supporté de façon coulissante par l'ensemble logement et s'étendant distalement depuis celui-ci et ayant une extrémité proximale et une extrémité distale ;
un guide d'onde à ultrasons (80) ayant une extrémité proximale et une extrémité distale est positionné à l'intérieur du tube externe ; et
l'effecteur terminal comprend une lame actionnée par ultrasons (79a) positionnée sur l'extrémité distale du guide d'onde.

6. Instrument chirurgical à ultrasons selon la revendication 5 comprenant en outre un élément de verrouillage supporté par l'ensemble logement, dans lequel l'élément de verrouillage (635) fournit une butée pour empêcher que le tube externe se déplace par translation par rapport à l'ensemble manche.

7. Instrument chirurgical à ultrasons selon la revendication 6, dans lequel l'élément de verrouillage tourne par rapport à l'ensemble manche.

8. Instrument chirurgical à ultrasons selon la revendication 6, dans lequel l'élément de verrouillage se déplace dans une direction normale à l'axe longitudinal.

9. Instrument chirurgical à ultrasons selon la revendication 5, dans lequel l'ensemble manche supporte de façon rotative le tube externe.

10. Instrument chirurgical à ultrasons selon la revendication 9, dans lequel le tube externe comprend des rainures (702).

11. Instrument chirurgical à ultrasons selon la revendication 5 comprenant en outre un ensemble d'éclairage.

12. Instrument chirurgical à ultrasons selon la revendication 5 comprenant en outre un ensemble contrepoids (820c), dans lequel l'ensemble contrepoids fournit un poids pour décaler le mouvement du tube externe.

13. Instrument chirurgical à ultrasons selon la revendication 1, dans lequel :
l'élément d'activation de puissance comprend un ensemble d'actionnement supporté de façon coulissante par un ensemble manche qui définit également un axe longitudinal ;
un tube externe est supporté par l'ensemble manche et s'étend distalement depuis celui-ci et possède une extrémité proximale et une extrémité distale ;
un guide d'onde à ultrasons ayant une extrémité proximale et une extrémité distale est positionné à l'intérieur du tube externe ; et
l'effecteur terminal comprend une lame actionnée par ultrasons positionnée sur l'extrémité distale du guide d'onde.

14. Instrument chirurgical à ultrasons selon la revendication 13, dans lequel l'ensemble manche supporte de façon coulissante le tube externe.
